# EUROPÄISCHE PATENTSCHRIFT

(11) **EP 1 525 879 B1**
(45) Veröffentlichungstag und Bekanntmachung des Hinweises auf die Patenterteilung: **20.09.2006**
(21) Anmeldenummer: 04105024.6
(22) Anmeldetag: 13.10.2004
(51) Int. Cl.: A61K 8/39, A61K 8/43, A61K 8/42, A61Q 17/02

(54) **Stabile kosmetische und dermatologische Zubereitungen**
Stable cosmetic and dermatological compositions
Compositions cosmétiques et dermatologiques stables

(30) Priorität: 24.10.2003 DE 10349679
(43) Veröffentlichungstag der Anmeldung: 27.04.2005
(73) Patentinhaber: Beiersdorf AG, 20253 Hamburg (DE)
(72) Erfinder: Kröpke, Rainer, 22869, Schenefeld (DE); Nielsen, Jens, 24558, Henstedt-Ulzburg (DE); Post, Katharina, 20359 Hamburg (DE)

(56) Entgegenhaltungen:
- EP-A- 1 275 370
- US-A- 5 980 871
- US-A1- 2002 022 660

## Beschreibung

Die vorliegende Erfindung betrifft PPG-1-PEG-9 Laurylglykolether in kosmetischen und/oder dermatologischen Zubereitungen sowie die Verwendung von PPG-1-PEG-9 Laurylglykolether in kosmetischen und/oder dermatologischen Zubereitungen.

Kosmetische und dermatologische Zubereitungen sind in der Regel hoch komplexe Mischungen aus unterschiedlichen Wirk-, Hilfs- und Zusatzstoffen. Diese Inhaltstoffe eines Kosmetikums beziehungsweise Dermatikums unterscheiden sich hinsichtlich ihrer chemischen und physikalischen Eigenschaften. Für den kosmetischen Chemiker oder Apotheker spielen daher neben physiologisch-toxikologischen Aspekten sowie der chemischen Reaktivität der Rezepturbestandteile einer Zubereitung, insbesondere deren Lösungs-, Emulgier- oder Dispergiereigenschaften eine entscheidende Rolle.

Das unterschiedliche Lösungs-, Emulgier- und Dispergiereigenschaften von kosmetischen und dermatologischen Rohstoffen basiert primär auf deren Polarität, d.h. der chemischen Natur der einzelnen Moleküle. In der Praxis unterscheidet der Fachmann grob zwischen wasserlöslichen, öllöslichen oder unlöslichen Substanzen, wobei der Übergang zwischen diesen drei Klassen natürlich fließend ist. Mit bloßem Auge homogen erscheinende flüssige Mischungen von Verbindungen mit unterschiedlicher Polarität werden in der Regel als Dispersionen (d.h. Mischungen eines oder mehrerer Feststoffe in einer Flüssigkeit), Emulsionen (d.h. Mischungen aus Flüssigkeiten mit stark unterschiedlicher Polarität, z.B. Wasser und Öl) und Lösungen (d.h. Stoffe, die in einer Flüssigkeit gelöst sind, wobei Stoff und Flüssigkeit entweder beide ähnlich polar oder unpolar sind) bezeichnet.

Um stabile flüssige Zubereitungen von Rohstoffen unterschiedlicher Polarität herstellen zu können, d.h. Zubereitungen, die sich über einen längeren Zeitraum nicht wieder (beispielsweise durch Phasentrennung) entmischen, werden den Formulierungen meist oberflächenaktive Verbindungen zugesetzt, d.h. Moleküle mit einem polaren und einem unpolarem Molekülteil. Hierzu zählen Tenside, Emulgatoren und Lösungsvermittler (Soulbilisatoren).

Als Solubilisation oder Solubilisierung bezeichnet man dabei das Löslichmachen eines in einer bestimmten Flüssigkeit unlöslichen Stoffes durch Zusatz von Lösungsvermittlern. In der kosmetisch-pharmazeutischen Praxis ist dabei die Überführung von wenig wasserlöslichen Substanzen in die wässrige Lösung ohne Veränderung ihrer chemischen Struktur von Bedeutung. Diese Art der Lösungsvermittlung kann mit Hilfe sogenannter hydrotroper Stoffe (d.h. Stoffe, die eine schwerlösliche Substanz in Gegenwart einer zweiten Komponente, die selbst kein Lösungsmittel darstellt, wasserlöslich macht z. B. ein- u. mehrwertige Alkohole, Ester, Ether, nichtionischen Tenside, Alkali- oder Erdalkalimetallsalze bestimmter organischer Säuren, Amide u. andere Stickstoff-haltige Verbindungen) erfolgen (Römpp, Chemie-Lexikon, Hrsg. J. Falbe, M. Regnitz, 9. Aufl., Georg Thieme Verlag, Stuttgart, New York, 1993).

Zur Solubilisierung von Parfümstoffen oder anderen unpolaren Verbindungen in wässrigen oder wässrig-alkoholischen kosmetischen und/oder dermatologischen Zubereitungen werden beispielsweise PEG-40 hydriertes Rizinusöl (PEG-40 hydrogenated Castor Oil) oder Laureth-4 eingesetzt. Derartige Lösungsvermittler haben jedoch den Nachteil, dass ihr Einsatz dazu führt, dass andere (wasserlösliche) Inhaltsstoffe der Zubereitung über kurz oder lang ausgefällt werden und zu einer Trübung der zunächst klaren Formulierung führen wodurch die Lager- bzw. Langzeitstabilität der Zubereitung beeinträchtigt wird. Derartige Probleme treten insbesondere bei dem Konservierungsmittel Polyaminopropylbiguanid und dem Insektenrepellent 3-(N-n-Butyl-N-acetyl-amino)-propionsäureethylester auf. Die Aufgabe der stabilen Einarbeitung solcher Verbindungen konnte nach dem Stande der Technik nur dadurch gelöst werden, dass die Konzentration dieser Inhaltsstoffe gering gehalten wurde, was insbesondere die Wirksamkeit von Wirkstoffen stark beeinträchtigte.

Es war daher die Aufgabe der vorliegenden Erfindung, die Nachteile des Standes der Technik zu beseitigen und kosmetische und/oder dermatologische Zubereitungen (insbesondere wässrige und wässrig-alkoholische Zubereitungen) zu entwickeln, in die sich insbesondere Polyaminopropylbiguanid und 3-(N-n-Butyl-N-acetyl-amino)-propionsäureethylester stabil einarbeiten lassen. Dabei sollte vor allem die Lager- bzw. Langzeitstabilität der Zubereitungen erhöht werden.

Ferner war es die Aufgabe der vorliegenden Erfindung, stabile (insbesondere lager- bzw. langzeitstabile) und bevorzugt transparente kosmetische und/oder dermatologische Zubereitungen auf Wasserbasis, Alkoholbasis und/oder wässrig-alkoholischer Basis zu entwickeln, die sowohl lipophile Bestandteile (insbesondere Parfümstoffe) als auch Polyaminopropylbiguanid und/oder 3-(N-n-Butyl-N-acetyl-amino)-propionsäureethylester enthalten.

Weiterhin war es die Aufgabe der vorliegenden Erfindung, das "Ausflocken" und "Eintrüben" transparenter kosmetischer und/oder dermatologischer Zubereitungen (insbesondere auf Wasserbasis, Alkoholbasis und/oder wässrig-alkoholischer Basis) zu verhindern. Insbesondere sollte das Ausfällen bzw. Ausflocken von Polyaminopropylbiguanid und 3-(N-n-Butyl-N-acetyl-amino)-propionsäureethylester aus den Zubereitungen (insbesondere aus Zubereitungen enthaltend lipophile Bestandteile wie Parfümstoffe) über einen längeren Zeitraum (> 1 Monat) verhindert werden.

Nicht zuletzt war es die Aufgabe der vorliegenden Erfindung, stabile (insbesondere lager- bzw. langzeitstabile) und bevorzugt transparente kosmetische und/oder dermatologische Zubereitungen auf Wasserbasis, Alkoholbasis und/oder wässrig-alkoholischer Basis zu entwickeln, die einen deutlich höheren Anteil an Polyaminopropylbiguanid und 3-(N-n-Butyl-N-acetyl-amino)-propionsäureethylester im Vergleich zum Stand der Technik enthalten.

Die Aufgaben werden für den Fachmann überraschend gelöst durch eine kosmetische und/oder dermatologische Zubereitung enthaltend eine Kombination aus
a) PPG-1-PEG-9 Laurylglykolether und
b) einem oder mehreren Wirkstoffen gewählt aus der Gruppe Polyaminopropylbiguanid und 3-(N-n-Butyl-N-acetyl-amino)-propionsäureethylester,
neben gegebenenfalls weiteren kosmetischen und/oder dermatologischern Wirk-, Hilfs- und Zusatzstoffen, sowie
durch die Verwendung von PPG-1-PEG-9 Laurylglykolether in kosmetischen und/oder dermatologischen Zubereitungen zur Erhöhung der Lagerstabilität von Wirkstoffen gewählt aus der Gruppe Polyaminopropylbiguanid und 3-(N-n-Butyl-N-acetyl-amino)-propionsäureethylester.

Der erfindungsgemäße PPG-1-PEG-9 Laurylglykolether (auch bekannt unter den Namen Polyoxyethylen (9) Polyoxypropylen (1) Laurylglykolether, CAS-Nr. 154248 -98-3) ist der ethoxyliert-propoxylierte Ether des Reaktionsproduktes aus Laurylepoxid und Ethylenglykol und wird unter dem Handelsnamen Eumulgin L® von der Firma Cognis als Lösungsvermittler angeboten.

Das erfindungsgemäße Polyaminopropylbiguanid (Imidodicarbonimidindiamid, N-(3-Aminopropyl)-Homopolymer, CAS-Nr. 120022-92-6) wird als Konservierungsmittel unter dem Handelsnamen Cosmocil CQ® von der Firma Zeneca sowie unter dem Handelsnamen Mikrokill® von der Firma Brooks angeboten.

Der erfindungsgemäße 3-(N-n-Butyl-N-acetyl-amino)-propionsäureethylester (Repellent 3535®) wird als Insektenrepellent gegen Stechmücken, Tsetsefliegen und Bremsen eingesetzt und ist bei der Fa. Merck erhältlich.

Der Stand der Technik kennt eine Vielzahl von Veröffentlichungen, in denen der Einsatz der Einzelkomponenten, beispielsweise von PPG-1-PEG-9 Laurylglykolether beschrieben werden:
- Das US-Patent US 5,424,070 beschreibt einen kosmetischen Stift enthaltend Eumulgin L. Dieses besteht jedoch aus PPG-2-Ceteareth-9.
- Die WO 00/40611 beschreibt eine schleimige kosmetische Zubereitung. Beispiel 3 offenbart eine alkoholische Zubereitung enthaltend PPG-1-PEG-9 Laurylglykolether, Konservierungsmittel und Parfüm. Nicht offenbart sind jedoch Art und Menge des Konservierungsmittels sowie des Parfüms.
- Die US 5,980.871 offenbart in Tabelle IV lipophile Zubereitungen enthaltend Eumulgin L
Diese Schriften konnten jedoch nicht den Weg zur vorliegenden Erfindung weisen.

Als wässrig bzw. wässrig-alkoholische Zubereitungen werden erfindungsgemäß Zubereitungen angesehen, die transparent oder transluzent sind und deren Anteil an lipophilen Bestandteilen 5,0 Gewichts-% nicht übersteigt.

Erfindungsgemäß vorteilhaft wird eine Kombination aus PPG-1-PEG-9 Laurylglykolether und Polyaminopropylbiguanid in den Zubereitungen eingesetzt bzw. verwendet.

Dabei ist es vorteilhaft im Sinne der vorliegenden Erfindung, wenn der Gehalt an PPG-1-PEG-9 Laurylglykolether von 0,05 bis 10,0 Gewichts-%, bevorzugt von 0,1 bis 5 Gewichts-% und besonders bevorzugt von 0,5 bis 2,5 Gewichts-%, jeweils bezogen auf das Gesamtgewicht der Zubereitung beträgt.

Ferner ist es in dieser Kombination vorteilhaft im Sinne der vorliegenden Erfindung, wenn der Gehalt an Polyaminopropylbiguanid von 0,01 bis 2,5 Gewichts-%, bevorzugt von 0,05 bis 1,5 Gewichts-% und besonders bevorzugt von 0,1 bis 1,0 Gewichts-%, jeweils bezogen auf das Gesamtgewicht der Zubereitung beträgt.

Erfindungsgemäß vorteilhaft wird eine Kombination aus G-1-PEG-9 Laurylglykolether und 3-(N-n-Butyl-N-acetyl-amino)-propionsäureethylester in den Zubereitungen eingesetzt bzw. verwendet.

Dabei ist es vorteilhaft im Sinne der vorliegenden Erfindung, wenn der Gehalt an PPG-1-PEG-9 Laurylglykolether von 0,01 bis 2,5 Gewichts-%, bevorzugt von 0,03 bis 1,5 Gewichts-% und besonders bevorzugt von 0,4 bis 0,75 Gewichts -%, jeweils bezogen auf das Gesamtgewicht der Zubereitung beträgt.

Ferner ist es in dieser Kombination vorteilhaft im Sinne der vorliegenden Erfindung, wenn der Gehalt an 3-(N-n-Butyl-N-acetyl-amino)-propionsäureethylester von 2,5 bis 60 Gewichts-%, bevorzugt von 5 bis 35 Gewichts-% und besonders bevorzugt von 10 bis 20 Gewichts-%, jeweils bezogen auf das Gesamtgewicht der Zubereitung beträgt.

Grundsätzlich vorteilhaft im Sinne der vorliegenden Erfindung sind Zubereitungen oder Verwendungen, die dadurch gekennzeichnet sind, dass PPG-1-PEG-9 Laurylglykolether in einer Konzentration von 0,05 bis 10,0 Gewichts -%, bezogen auf das Gesamtgewicht der Zubereitung, eingesetzt wird.

Grundsätzlich vorteilhaft im Sinne der vorliegenden Erfindung sind Zubereitungen oder Verwendungen, die dadurch gekennzeichnet sind, dass das Gewichtsverhältnis von PPG-1-PEG-9 Laurylglykolether zu Wirkstoffen, gewählt aus der Gruppe Polyaminopropylbiguanid und 3-(N-n-Butyl-N-acetyl-amino)-propionsäureethylester, von 5 : 1 bis 1 : 20 beträgt.

Erfindungsgemäß vorteilhaft handelt es sich bei der erfindungsgemäßen Zubereitung oder Verwendung um eine wässrige oder wässrig-alkoholische Zubereitung.

Insbesondere ist es erfindungsgemäß von Vorteil, wenn der Gesamtgehalt an lipophilen Substanzen von 0,05 bis 5,0 Gewichts-%, bezogen auf das Gesamtgewicht der Zubereitung, beträgt.

Die Wasserphase der erfindungsgemäßen Zubereitungen kann vorteilhaft übliche kosmetische Hilfsstoffe enthalten, wie beispielsweise Alkohole, insbesondere solche niedriger C-Zahl, Diole oder Polyole niedriger C-Zahl sowie deren Ether, vorzugsweise Propylenglykol, Glycerin, Butylenglykol, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, Polymere, Schaumstabilisatoren, Elektrolyte sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole [von der Fa. Bf. Goodrich], beispielsweise Carbopole der Typen 980, 981, 1382, 2984, 5984, ETD 2020, ETD 2050, Ultrez 10, jeweils einzeln oder in Kombination.

Es ist erfindungsgemäß bevorzugt, wenn ein oder mehrere Alkohole gewählt werden aus der Gruppe Ethanol, n-Propanol und/oder Isopropanol. Erfindungsgemäß besonders bevorzugt sind Zubereitungen die Ethanol als Alkohol enthalten.

Erfindungsgemäß vorteilhaft beträgt die Viskosität der erfindungsgemäßen Zubereitungen von bis mPas und bevorzugt von 1 bis 100 mPas (gemessen bei 25°C, einer Scherrate von 10 Pa/s mit dem Gerät Haake VT 02 etc.)

Die Zubereitungen der vorliegenden Erfindung können erfindungsgemäß vorteilhaft weitere kosmetische oder dermatologische Wirk-, Hilfs- und Zusatzstoffe enthalten:

So können die erfindungsgemäßen Zubereitungen vorteilhaft weitere Repellentien zum Schutz vor Mücken, Zecken und Spinnen und dergleichen enthalten. Vorteilhaft sind z. B. N,N-Diethyl-3-methylbenzamid (Handelsbezeichnung: Meta-delphene, "DEET"), Dimethylphtalat (Handelsbezeichnung: Palatinol M, DMP), 1-Piperidincarbonsäure-2-(2-hydroxyethyl)-1-methylpropylester.
Die kosmetischen und dermatologischen Zubereitungen gemäß der Erfindung können kosmetische Hilfsstoffe enthalten, wie sie üblicherweise in solchen Zubereitungen verwendet werden, z. B. weitere Konservierungsmittel, Konservierungshelfer, Komplexbildner, Bakterizide, Parfüme, Substanzen zum Verhindern oder Steigern des Schäumens, Farbstoffe, Pigmente, die eine färbende Wirkung haben oder andere übliche Bestandteile einer kosmetischen oder dermatologischen Formulierung wie Polymere, Schaumstabilisatoren, Elektrolyte, organische Lösungsmittel oder Silikonderivate.

Die erfindungsgemäße Zubereitung kann erfindungsgemäß vorteilhaft ein oder mehrere zusätzliche Konservierungsstoffe enthalten. Vorteilhafte Konservierungsstoffe im Sinne der vorliegenden Erfindung sind beispielsweise Formaldehydabspalter (wie z. B. DMDM Hydantoin, welches beispielsweise unter der Handelsbezeichnung Glydant™ von der Fa. Lonza erhältlich ist), lodopropylbutylcarbamate (z. B. die unter den Handelsbezeichnungen Glycacil-L, Glycacil-S von der Fa. Lonza erhältlichen und/oder Dekaben LMB von Jan Dekker), Parabene (d. h. p-Hydroxybenzoesäurealkylester, wie Methyl-, Ethyl-, Propyl- und/oder Butylparaben), Phenoxyethanol, Ethanol, Benzoesäure und dergleichen mehr. Üblicherweise umfaßt das Konservierungssystem erfindungsgemäß ferner vorteilhaft auch Konservierungshelfer, wie beispielsweise Octoxyglycerin, Glycine Soja etc. Die nachfolgende Tabelle gibt einen Überblick über einige erfindungsgemäß vorteilhafte Konservierungsstoffe:

| | | | |
|---|---|---|---|
| E 200 | Sorbinsäure | E 227 | Calciumhydrogensulfit |
| E 201 | Natriumsorbat | E 228 | Kaliumhydrogensulfit) |
| E 202 | Kaliumsorbat | E 230 | Biphenyl (Diphenyl) |
| E 203 | Calciumsorbat | E 231 | Orthophenylphenol |
| E 210 | Benzoesäure | E 232 | Natriumorthophenylphenolat |
| E 211 | Natriumbenzoat | E 233 | Thiabendazol |
| E 212 | Kaliumbenzoat | E 235 | Natamycin |
| E 213 | Calciumbenzoat | E 236 | Ameisensäure |
| E 214 | p-Hydroxybenzoesäureethylester | E 237 | Natriumformiat |
| E 215 | p-Hydroxybenzoesäureethylester-Na-Salz | E 238 | Calciumformiat |
| E 216 | p-Hydroxybenzoesäure-n-propylester | E 239 | Hexamethylentetramin |
| E 217 | p-Hydroxybenzoesäure-n-propylester-Na-Salz | E 249 | Kaliumnitrit |
| E 218 | p-Hydroxybenzoesäuremethylester | E 250 | Natriumnitrit |
| E 219 | p-Hydroxybenzoesäuremethylester-Na-Salz | E 251 | Natriumnitrat |
| E 220 | Schwefeldioxid | E 252 | Kaliumnitrat |
| E 221 | Natriumsulfit | E 280 | Propionsäure |
| E 222 | Natriumyhdrogensulfit | E 281 | Natriumpropionat |
| E 223 | Natriumdisulfit | E 282 | Calciumpropionat |
| E 224 | Kaliumdisulfit | E 283 | Kaliumpropionat |
| E 226 | Calciumsulfit | E 290 | Kohlendioxid |

Ferner vorteilhaft sind in der Kosmetik gebräuchliche Konservierungsmittel oder Konservierungshilfsstoffe, wie Dibromdicyanobutan (2-Brom-2-brommethylglutarodinitril), Phenoxyethanol, 3-lod-2-propinylbutylcarbamat, 2-Brom-2-nitro-propan-1,3-diol, Imidazolidinylharnstoff, 5-Chlor-2-methyl-4-isothiazolin-3-on, 2-Chloracetamid, Benzalkoniumchlorid, Benzylalkohol, Salicylsäure und Salicylate.

Es ist dabei erfindungsgemäß besonders bevorzugt, wenn als weitere Konservierungsstoffe lodopropylbutylcarbamate, Parabene (Methyl-, Ethyl-, Propyl- und/oder Butylparaben) und/oder Phenoxyethanol eingesetzt werden.

Vorteilhafte Komplexbildner im Sinne der vorliegenden Erfindung sind beispielsweise EDTA, [S,S]-Ethylendiamindisuccinat (EDDS), welches beispielsweise unter der Handelsbezeichnung Octaquest von der Fa. Octel erhältlich ist, Pentanatrium-Ethylendiamintetramethylenphosphonat, welches z. B. unter dem Handelsnamen Dequest 2046 von der Fa. Monsanto erhältlich ist und/oder Iminodibersteinsäure, welche u. a. von der Fa. Bayer AG unter den Handelsnamen Iminodisuccinat VP OC 370 (ca. 30% ige Lösung) und Baypure CX 100 fest erhältlich ist.

Vorteilhafte Moisturizer im Sinne der vorliegenden Erfindung sind beispielsweise Glycerin, Milchsäure und/oder Lactate, insbesondere Natriumlactat, Butylenglykol, Propylenglykol, Biosaccaride Gum-1, Glycine Soja, Ethylhexyloxyglycerin, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel®1000 von der Gesellschaft SOLABIA S.A. erhältlich ist.

Als Moisturizer werden Stoffe oder Stoffgemische bezeichnet, welche kosmetischen oder dermatologischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen. Moisturizer können vorteilhaft auch als Antifaltenwirkstoffe zur Prophylaxe und Behandlung kosmetischer oder dermatologischer Hautveränderungen, wie sie z. B. bei der Hautalterung auftreten, verwendet werden.

Besonders vorteilhafte Zubereitungen werden ferner erhalten, wenn als Zusatz- oder Wirkstoffe Antioxidantien eingesetzt werden. Erfindungsgemäß enthalten die Zubereitungen vorteilhaft eines oder mehrere Antioxidantien. Als günstige, aber dennoch fakultativ zu verwendende Antioxidantien können alle für kosmetische und/oder dermatologische Anwendungen geeigneten oder gebräuchlichen Antioxidantien verwendet werden.

Besonders vorteilhaft im Sinne der vorliegenden Erfindung können wasserlösliche Antioxidantien eingesetzt werden, wie beispielsweise Vitamine, z. B. Ascorbinsäure und deren Derivate.

Bevorzugte Antioxidantien sind ferner Vitamin E und dessen Derivate sowie Vitamin A und dessen Derivate.

Die Menge der Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Sofern Vitamin A bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Es ist insbesondere vorteilhaft, wenn die kosmetischen Zubereitungen gemäß der vorliegenden Erfindung kosmetische oder dermatologische Wirkstoffe enthalten, wobei bevorzugte Wirkstoffe Antioxidantien sind, welche die Haut vor oxidativer Beanspruchung schützen können.

Weitere vorteilhafte Wirkstoffe im Sinne der vorliegenden Erfindung sind natürliche Wirkstoffe und/oder deren Derivate, wie z. B. alpha-Liponsäure, Phytoen, D-Biotin, Coenzym Q10, alpha-Glucosylrutin, Carnitin, Carnosin, natürliche und/oder synthetische Isoflavonoide, Kreatin, Kreatinin, Taurin und/oder β-Alanin sowie 8-Hexadecen-1,16-dicarbonsäure (Dioic acid, CAS-Nummer 20701-68-2; vorläufige INCI-Bezeichnung Octadecendioic acid).

Die erfindungsgemäße Zubereitung kann auch in Form eines Schaumes eingesetzt bzw. aufgeschäumt werden.

Aufschäumbare erfindungsgemäße Zubereitungen enthalten ein oder mehrere Tenside. Diese können aus der Gruppe der anionischen, kationischen, nichtionischen oder amphotheren Tenside gewählt werden.

Vorteilhafte anionische Tenside im Sinne der vorliegenden Erfindung sind Acylaminosäuren und deren Salze, wie
■ Acylglutamate, insbesondere Natriumacylglutamat
■ Sarcosinate, beispielsweise Myristoyl Sarcosin, TEA-lauroyl Sarcosinat, Natriumlauroylsarcosinat und Natriumcocoylsarkosinat,

Sulfonsäuren und deren Salze, wie
■ Acylisethionate, z.B. Natrium-/ Ammoniumcocoylisethionat,
■ Sulfosuccinate, beispielsweise Dioctylnatriumsulfosuccinat, Dinatriumlaurethsulfosuccinat, Dinatriumlaurylsulfosuccinat und Dinatriumundecylenamido MEA-Sulfosuccinat, Dinatrium PEG-5 Laurylcitratsulfosuccinat und Derivate,
sowie Schwefelsäureester, wie
■ Alkylethersulfat, beispielsweise Natrium-, Ammonium-, Magnesium-, MIPA-, TIPA-Laurethsulfat, Natriummyrethsulfat und Natrium C₁₂₋₁₃ Parethsulfat,
■ Alkylsulfate, beispielsweise Natrium-, Ammonium- und TEA- Laurylsulfat.

Weitere vorteilhafte anionische Tenside sind
■ Taurate, beispielsweise Natriumlauroyltaurat und Natriummethylcocoyltaurat,
■ Ether-Carbonsäuren, beispielsweise Natriumlaureth-13 Carboxylat und Natrium PEG-6 Cocamide Carboxylat, Natrium PEG-7-Olivenöl-Carboxylat
■ Phosphorsäureester und Salze, wie beispielsweise DEA-Oleth-10 Phosphat und Dilaureth-4 Phosphat,
■ Alkylsulfonate, beispielsweise Natriumcocosmonoglyceridsulfat, Natrium C₁₂₋₁₄ Olefinsulfonat, Natriumlaurylsulfoacetat und Magnesium PEG-3 Cocamidsulfat,
■ Acylglutamate wie Di-TEA-palmitoylaspartat und Natrium Caprylic/ Capric Glutamat,
■ Acylpeptide, beispielsweise Palmitoyl hydrolysiertes Milchprotein, Natrium Cocoyl hydrolysiertes Soja Protein und Natrium-/ Kalium Cocoyl hydrolysiertes Kollagen
sowie Carbonsäuren und Derivate, wie
■ beispielsweise Laurinsäure, Aluminiumstearat, Magnesiumalkanolat und Zinkundecylenat,
■ Ester-Carbonsäuren, beispielsweise Calciumstearoyllactylat, Laureth-6 Citrat und Natrium PEG-4 Lauramidcarboxylat,
■ Alkylarylsulfonate.

Vorteilhafte kationische Tenside im Sinne der vorliegenden Erfindung sind quaternäre Tenside. Quaternäre Tenside enthalten mindestens ein N-Atom, das mit 4 Alkyl- oder Arylgruppen kovalent verbunden ist. Vorteilhaft sind beispielsweise Alkylbetain, Alkylamidopropylbetain und Alkylamidopropylhydroxysultain.
Weitere vorteilhafte kationische Tenside im Sinne der vorliegenden Erfindung sind ferner
■ Alkylamine,
■ Alkylimidazole und
■ ethoxylierte Amine
und insbesondere deren Salze.

Vorteilhafte amphotere Tenside im Sinne der vorliegenden Erfindung sind Acyl-/dialkylethylendiamine, beispielsweise Natriumacylamphoacetat, Dinatriumacylamphodipropionat, Dinatriumalkylamphodiacetat, Natriumacylamphohydroxypropylsulfonat, Dinatriumacylamphodiacetat, Natriumacylamphopropionat, und N-Kokosfettsäureamidoethyl-N-hydroxyethylglycinat Natriumsalze.

Weitere vorteilhafte amphotere Tenside sind N-Alkylaminosäuren, beispielsweise Aminopropylalkylglutamid, Alkylaminopropionsäure, Natriumalkylimidodipropionat und Lauroamphocarboxyglycinat.

Vorteilhafte nicht-ionische Tenside im Sinne der vorliegenden Erfindung sind
■ Alkanolamide, wie Cocamide MEA/ DEA/ MIPA,
■ Ester, die durch Veresterung von Carbonsäuren mit Ethylenoxid, Glycerin, Sorbitan oder anderen Alkoholen entstehen,
■ Ether, beispielsweise ethoxylierte Alkohole, ethoxyliertes Lanolin, ethoxylierte Polysiloxane, propoxylierte POE Ether und Alkylpolyglycoside wie Laurylglucosid, Decylglycosid und Cocoglycosid.
Weitere vorteilhafte nicht-ionische Tenside sind Alkohole und Aminoxide, wie Cocoamidopropylaminoxid.

Besonders vorteilhaft im Sinne der Erfindung ist es, nichionische Tenside zum aufschäumen zu verwenden. Beispielsweise vorteilhaft ist es, Gemische aus Stearylglucosid und Cetylglucosid zu verwenden. Solche Mischungen sind im Handel beispielsweise unter der Warenbezeichnung Tego® Care CG 90 (Cetearylpolyglucosid) von der Gesellschaft Th.Goldschmidt KG erhältlich.

Die erfindungsgemäße Zubereitung kann ein oder mehrere Tenside in einer Konzentration von 0,01 bis 20 Gewichts-% und besonders bevorzugt von 0,1 bis 5 Gewichts-%, jeweils bezogen auf das Gesamtgewicht der Zubereitung einzusetzen.

Es ist vorteilhaft im Sinne der vorliegenden Erfindung, wenn die erfindungsgemäße Zubereitung zusätzlich mindestens eine UV-A-Filtersubstanz und/oder mindestens eine UV-B-Filtersubstanz enthält.

Vorteilhafte weitere UV-Filtersubstanzen im Sinne der vorliegenden Erfindung sind sulfonierte, wasserlösliche UV-Filter, wie z. B.:
- Phenylen-1,4-bis-(2-benzimidazyl)-3,3'-5,5'-tetrasulfonsäure und ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz mit der INCI-Bezeichnung Disodium Phenyl Dibenzimidazol Tetrasulfonat (CAS-Nr.: 180898-37-7), welches beispielsweise unter der Handelsbezeichnung Neo Heliopan AP bei Haarmann & Reimer erhältlich ist;
- Salze der 2-Phenylbenzimidazol-5-sulfonsäure, wie ihr Natrium-, Kalium- oder ihr Triethanolammonium-Salz sowie die Sulfonsäure selbst mit der INCI Bezeichnung Phenylbenzimidazole Sulfonsäure (CAS.-Nr. 27503-81-7), welches beispielsweise unter der Handelsbezeichnung Eusolex 232 bei Merck oder unter Neo Heliopan Hydro bei Haarmann & Reimer erhältlich ist;
- 1,4-di(2-oxo-10-Sulfo-3-bornylidenmethyl)-Benzol (auch: 3,3'-(1,4-Phenylendimethylene)-bis-(7,7-dimethyl-2-oxo-bicyclo-[2.2.1]hept-1-ylmethan Sulfonsäure) und dessen Salze (besonders die entprechenden 10-Sulfato-verbindungen, insbesondere das entsprechende Natrium-, Kalium- oder Triethanolammonium-Salz), das auch als Benzol-1,4-di(2-oxo-3-bornylidenmethyl-10-sulfonsäure) bezeichnet wird. Benzol-1,4-di(2-oxo-3-bornylidenmethyl-10-sulfonsäure) hat die INCI-Bezeichnung Terephtalidene Dicampher Sulfonsäure (CAS.-Nr.: 90457-82-2) und ist beispielsweise unter dem Handelsnamen Mexoryl SX von der Fa. Chimex erhältlich;
- Sulfonsäure-Derivate des 3-Benzylidencamphers, wie z. B. 4-(2-Oxo-3-bornylidenmethyl)benzolsulfonsäure, 2-Methyl-5-(2-oxo-3-bornylidenmethyl)sulfonsäure und deren Salze.

Vorteilhafte UV-Filtersubstanzen im Sinne der vorliegenden Erfindung sind ferner sogenannte Breitbandfilter, d.h. Filtersubstanzen, die sowohl UV-A- als auch UV-B-Strahlung absorbieren.

Vorteilhafte Breitbandfilter oder UV-B-Filtersubstanzen sind beispielsweise Triazinderivate, wie z. B.
- 2,4-Bis-{[4-(2-Ethyl-hexyloxy)-2-hydroxy]-phenyl}-6-(4-methoxyphenyl)-1,3,5-triazin (INCI: Bis-Ethylhexyloxylphenol Methoxyphenyl Triazin), welches unter der Handelsbezeichnung Tinosorb® S bei der CIBA-Chemikalien GmbH erhältlich ist;
- Dioctylbutylamidotriazon (INCI: Dioctylbutamidotriazone), welches unter der Handelsbezeichnung UVASORB HEB bei Sigma 3V erhältlich ist;
- 4,4',4"-(1,3,5-Triazin-2,4,6-triyltriimino)-tris-benzoësäure-tris(2-ethylhexylester), auch: 2,4,6-Tris-[anilino-(p-carbo-2'-ethyl-1'-hexyloxy)]-1,3,5-triazin (INCI: Octyl Triazone), welches von der BASF Aktiengesellschaft unter der Warenbezeichnung UVINUL® T 150 vertrieben wird;
- 2-[4,6-Bis(2,4-dimethylphenyl)-1,3,5-triazin-2-yl]-5-(octyloxy)phenol (CAS Nr.: 2725-22-6).

Ein vorteilhafter Breitbandfilter im Sinne der vorliegenden Erfindung ist auch das 2,2'-Methylen-bis-(6-(2H-benzotriazol-2-yl)-4-(1,1,3,3-tetramethylbutyl)-phenol), welches unter der Handelsbezeichnung Tinosorb® M bei der CIBA-Chemikalien GmbH erhältlich ist.

Vorteilhafter Breitbandfilter im Sinne der vorliegenden Erfindung ist ferner das 2-(2H-benzotriazol-2-yl)-4-methyl-6-[2-methyl-3-[1,3,3,3-tetramethyl-1-[(trimethylsilyl )oxy]d isiloxanyl]propyl]-phenol (CAS-Nr.: 155633-54-8) mit der INCI-Bezeichnung Drometrizole Trisiloxane.

Vorteilhafte wasserlösliche Filtersubstanzen sind z. B.:
Sulfonsäure-Derivate des 3-Benzylidencamphers, wie z. B. 4-(2-Oxo-3-bornylidenmethyl)-benzolsulfonsäure, 2-Methyl-5-(2-oxo-3-bornylidenmethyl)sulfonsäure und deren Salze.

Eine weiterere erfindungsgemäß vorteilhaft zu verwendende Lichtschutzfiltersubstanz ist das Ethylhexyl-2-cyano-3,3-diphenylacrylat (Octocrylen), welches von BASF unter der Bezeichnung Uvinul® N 539 T erhältlich ist.

Erfindungsgemäß vorteilhafte UV -Filter finden sich auch unter den Benzoxazol -Derivaten. Ein erfindungsgemäß bevorzugtes Benzoxazol-Derivat ist das 2,4-bis-[5-1 (dimethylpropyl)benzoxazol-2-yl-(4-phenyl)-imino]-6-(2-ethylhexyl)-imino-1,3,5-triazin mit der CAS Nr. 288254-16-0, welches bei 3V Sigma unter der Handelsbezeichnung Uvasorb® K2A erhältlich ist.

Erfindungsgemäß vorteilhafte UV-Filter finden sich auch unter den Hydroxybenzophenonen. Ein besonders vorteilhaftes Hydroxybenzophenon im Sinne der vorliegenden Erfindung ist der 2-(4'-Diethylamino-2'-hydoxybenzoyl)-benzoesäurehexylester (auch: Aminobenzophenon), welcher unter dem Namen Uvinul A Plus bei der Fa. BASF erhältlich ist.

Die Zusammensetzungen enthalten gemäß der Erfindung außer den vorgenannten Substanzen gegebenenfalls die in der Kosmetik üblichen Zusatzstoffe, beispielsweise Parfüm, Farbstoffe, antimikrobielle Stoffe, rückfettende Agentien, Komplexierungs- und Sequestrierungsagentien, Perlglanzagentien, Pflanzenextrakte, Vitamine, Wirkstoffe, Bleichmittel, Selbstbräuner (z.B. DHA), Bakterizide, Pigmente, die eine färbende Wirkung haben, Verdickungsmittel, weichmachende, anfeuchtende und/oder feuchthaltende Substanzen, oder andere übliche Bestandteile einer kosmetischen oder dermatologischen Formulierung wie Polymere, Schaumstabilisatoren, Elektrolyte, organische Lösemittel oder Silikonderivate.

Die Zubereitungen gemäß der vorliegenden Erfindung können mittels eines Sprays oder Pumpschäumers auf die Körperoberflächen aufgetragen werden. Ferner können sie in Form eines getränkten Tuches oder Schwammes angewendet werden.

Die Zubereitungen gemäß der vorliegenden Erfindung können erfindungsgemäß zur Reinigung und/oder Pflege der Haut, der Haare und der Nägel eingesetzt werden sowie zum Schutz vor Insekten, Mücken, Spinnen und anderen Parasiten.

Ferner können sie zur Behandlung und Pflege von Gegenständen des täglichen Bedarfs eingesetzt werden.

### Vergleichsversuch

Der überraschende Effekt einer transparenten Zubereitung der vorliegenden Erfindung lässt sich *beispielsweise* durch den folgenden Nachweis belegen: Es wurden 3 wässrige Zubereitungen hergestellt, deren Zusammensetzung mit Ausnahme des Lösungsvermittlers identisch waren. Während die Zubereitung mit dem herkömmlichen Lösungsvermittler PEG-40 Hydrogenated Castor Oil zu einer Trübung der Polyaminopropylbiguanid-haltigen Zusammensetzung führte, blieben die erfindungsgemäßen Zubereitungen V1 und V2 transparent.

| **Wässrige Zubereitung** | **V 1** | **V 2** | **Vergleich** |
|---|---|---|---|
| PPG-1-PEG-9-Laurylglycolether | 1,0 | 0,5 | - |
| PEG-40 Hydrogenated Castor Oil | - | - | 1,0 |
| Polyaminopropylbiguanid | 1,0 | 1,0 | 1,0 |
| Ethanol | 5 | 5 | 5 |
| Methylparaben | 0,1 | 0,1 | 0,1 |
| Glycerin | 10 | 10 | 10 |
| Wasser | ad 100 | ad 100 | ad 100 |
| **Aussehen** | **Transparent** | **Transparent** | **Trübe** |

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

### Beispiele

**Pumpspray mit Repellent**

| | **1** | **2** | **3** | **4** | **5** |
|---|---|---|---|---|---|
| PPG-1-PEG-9-Laurylglycolether | 1,0 | 2,5 | 0,4 | 0,75 | 5,0 |
| Cyclomethicon | 0,5 | - - - | - - - | - - - | 1,5 |
| Ethylbutylacetylaminopropionat | 2 | 10 | 10 | 20 | 40 |
| Parfum | q,s, | q,s, | q,s, | q,s, | q,s, |
| Ethanol | 15 | 20 | 45 | 40 | 20 |
| Methylparaben | 0,4 | 0,1 | - - - | 0,3 | 0,4 |
| Meeresextrakt | - - - | - - - | - - - | - - - | - - - |
| Aloe Vera Extrakt | 0,1 | - - - | 0,1 | - - - | - - - |
| Hammamelis-Extrakt | 0,1 | - - - | 0,1 | 5 | 0,5 |
| Glycerin | 5 | 10 | 10 | 15 | 7,5 |
| Benzophenon-2 | 0,05 | - - - | 0,02 | - - - | - - - |
| modifizierte Stärke | - - - | 2,5 | - - - | 0,15 | - - |
| Wasser | ad 100 | ad 100 | ad 100 | ad 100 | ad 100 |

| **Wässrige Zubereitung** | **1** | **2** | **3** | **4** | **5** |
|---|---|---|---|---|---|
| PPG-1-PEG-9-Laurylglycolether | 1,0 | 0,5 | 0,4 | 0,75 | 3,0 |
| Polyaminopropylbiguanid | 1,0 | 2,0 | 0,2 | 0,1 | 0,5 |
| Parfum | q,s, | q,s, | q,s, | q,s, | q,s, |
| Ethanol | - | - | 5 | 10 | - |
| Methylparaben | 0,4 | 0,1 | - - - | 0,3 | 0,4 |
| Panthenol | - - - | 0,05 | 0,5 | - - - | - - - |
| Aloe Vera Extrakt | 0,1 | - - - | 0,1 | - - - | - - - |
| Hammamelis-Extrakt | 0,1 | - - - | 0,1 | 5 | 0,5 |
| Glycerin | 5 | 10 | 10 | 15 | 7,5 |
| Benzophenon-2 | 0,05 | - - - | 0,02 | - - - | - - - |
| EDTA | - - - | 0,5 | - - - | 0,15 | - - |
| Wasser | ad 100 | ad 100 | ad 100 | ad 100 | ad 100 |

## Patentansprüche

1. Kosmetische und/oder dermatologische Zubereitung enthaltend eine Kombination aus
a) PPG-1-PEG-9 Laurylglykolether und
b) einem oder mehreren Wirkstoffen gewählt aus der Gruppe Polyaminopropylbiguanid und 3-(N-n-Butyl-N-acetyl-amino)-propionsäureethylester, neben gegebenenfalls weiteren kosmetischen und/oder dermatologischern Wirk-, Hilfs- und Zusatzstoffen.

2. Verwendung von PPG-1-PEG-9 Laurylglykolether in kosmetischen und/oder dermatologischen Zubereitungen zur Erhöhung der Lagerstabilität von Wirkstoffen gewählt aus der Gruppe Polyaminopropylbiguanid und 3-(N-n-Butyl-N-acetyl-amino)-propionsäureethylester.

3. Zubereitung nach Anspruch 1 oder Verwendung nach Anspruch 2, **dadurch gekennzeichnet, dass** es sich um eine Kombination aus PPG-1-PEG-9 Laurylglykolether und Polyaminopropylbiguanid handelt.

4. Zubereitung nach Anspruch 1 oder Verwendung nach Anspruch 2, **dadurch gekennzeichnet, dass** es sich um eine Kombination aus G-1-PEG-9 Laurylglykolether und 3-(N-n-Butyl-N-acetyl-amino)-propionsäureethylester handelt.

5. Zubereitung oder Verwendung nach einem der vorhergehenden Ansprüche, **dadurch gekennzeichnet, dass** PPG-1-PEG-9 Laurylglykolether in einer Konzentration von 0,05 bis 10 Gewichts-%, bezogen auf das Gesamtgewicht der Zubereitung, eingesetzt wird.

6. Zubereitung oder Verwendung nach einem der vorhergehenden Ansprüche, **dadurch gekennzeichnet, dass** das Gewichtsverhältnis von PPG-1-PEG-9 Laurylglykolether zu Wirkstoffen, gewählt aus der Gruppe Polyaminopropylbiguanid und 3-(N-n-Butyl-N-acetyl-amino)-propionsäureethylester, von 5 : 1 bis 1 : 20 beträgt.

7. Zubereitung oder Verwendung nach einem der vorhergehenden Ansprüche, **dadurch gekennzeichnet, dass** das die Zubereitung eine wässrige oder wässmg-alkoholische Zubereitung ist.

8. Zubereitung oder Verwendung nach einem der vorhergehenden Ansprüche, **dadurch gekennzeichnet, dass** der Gesamtgehalt an lipophilen Substanzen von 0,1 bis 5,0 Gewichts-%, bezogen auf das Gesamtgewicht der Zubereitung, beträgt.

## Claims

1. Cosmetic and/or dermatological composition comprising a combination of
a) PPG-1-PEG-9 lauryl glycol ether and
b) one or more active ingredients chosen from the group polyaminopropylbiguanide and ethyl 3-(N-n-butyl-N-acetylamino)propionate, besides, if appropriate, further cosmetic and/or dermatological active ingredients, auxiliaries and additives.

2. Use of PPG-1-PEG-9 lauryl glycol ether in cosmetic and/or dermatological compositions for increasing the storage stability of active ingredients chosen from the group polyaminopropylbiguanide and ethyl 3-(N-n-butyl-N-acetylamino)propionate.

3. Composition according to Claim 1 or use according to Claim 2, **characterized in that** it is a combination of PPG-1-PEG-9 lauryl glycol ether and polyaminopropylbiguanide.

4. Composition according to Claim 1 or use according to Claim 2, **characterized in that** it is a combination of PPG-1-PEG-9 lauryl glycol ether and ethyl 3-(N-n-butyl-N-acetylamino)propionate.

5. Composition or use according to one of the preceding claims, **characterized in that** PPG-1-PEG-9 lauryl glycol ether is used in a concentration of from 0.05 to 10% by weight, based on the total weight of the composition.

6. Composition or use according to one of the preceding claims, **characterized in that** the weight ratio of PPG-1-PEG-9 lauryl glycol ether to active ingredients chosen from the group polyaminopropylbiguanide and ethyl 3-(N-n-butyl-N-acetylamino)propionate is from 5:1 to 1:20.

7. Composition or use according to one of the preceding claims, **characterized in that** the composition is an aqueous or aqueous-alcoholic composition.

8. Composition or use according to one of the preceding claims, **characterized in that** the total content of lipophilic substances is from 0.1 to 5.0% by weight, based on the total weight of the composition.

## Revendications

1. Préparation cosmétique et/ou dermatologique contenant une association de
a) PPG-1-PEG-9 laurylglycoléther et
b) une ou plusieurs substances actives choisies dans le groupe constitué par le polyaminopropylbiguanide et le 3-(N-n-butyl-N-acétylamino)propionate d'éthyle, en plus éventuellement d'autres actifs, adjuvants et additifs cosmétiques et/ou dermatologiques.

2. Utilisation de PPG-1-PEG-9 laurylglycoléther dans des préparations cosmétiques et/ou dermatologiques pour augmenter la stabilité au stockage de substances actives choisies dans le groupe constitué par le polyaminopropylbiguanide et le 3-(N-n-butyl-N-acétylamino)propionate d'éthyle.

3. Préparation selon la revendication 1 ou utilisation selon la revendication 2, **caractérisée en ce qu'**il s'agit d'une association de PPG-1-PEG-9 laurylglycoléther et polyaminopropylbiguanide.

4. Préparation selon la revendication 1 ou utilisation selon la revendication 2, **caractérisée en ce qu'**il s'agit d'une association de PPG-1-PEG-9 laurylglycoléther et 3-(N-n-butyl-N-acétylamino)propionate d'éthyle.

5. Préparation ou utilisation selon l'une quelconque des revendications précédentes, **caractérisée en ce que** le PPG-1-PEG-9 laurylglycoléther est utilisé à une concentration de 0,05 à 10 % en poids, par rapport au poids total de la préparation.

6. Préparation ou utilisation selon l'une quelconque des revendications précédentes, **caractérisée en ce que** le rapport pondéral du PPG-1-PEG-9 laurylglycoléther aux substances actives choisies dans le groupe constitué par le polyaminopropylbiguanide et le 3-(N-n-butyl-N-acétylamino)propionate d'éthyle, va de 5:1 à 1:20.

7. Préparation ou utilisation selon l'une quelconque des revendications précédentes, **caractérisée en ce que** la préparation est une préparation aqueuse ou aqueuse-alcoolique.

8. Préparation ou utilisation selon l'une quelconque des revendications précédentes, **caractérisée en ce que** le poids total de substances lipophiles va de 0,1 à 5,0 % en poids, par rapport au poids total de la préparation.
